# EUROPEAN PATENT SPECIFICATION

(11) **EP 3 440 015 B1**
(45) Date of publication and mention of the grant of the patent: **14.07.2021**
(21) Application number: 17715111.5
(22) Date of filing: 31.03.2017
(51) Int. Cl.: C01F 5/24, A61K 31/496, H01L 51/52

(54) **HIGHLY POROUS MAGNESIUM CARBONATE AND METHOD OF PRODUCTION THEREOF**
HOCHPORÖSES MAGNESIUMCARBONAT UND VERFAHREN ZUR HERSTELLUNG DAVON
CARBONATE DE MAGNÉSIUM HAUTEMENT POREUX ET SON PROCÉDÉ DE PRODUCTION

(30) Priority: 04.04.2016 SE 1650452
(43) Date of publication of application: 13.02.2019
(73) Proprietor: Disruptive Materials AB, 751 83 Uppsala (SE)
(72) Inventor: CHEUNG, Ocean, 182 67 Stockholm (SE); ZHANG, Peng, 752 71 Uppsala (SE); GUSTAFSSON, Simon, 752 34 Uppsala (SE); FRYKSTRAND ÅNGSTRÖM, Sara, 191 38 Sollentuna (SE); STRØMME, Maria, 756 51 Uppsala (SE)
(74) Representative: Brann AB
(86) International application number: PCT/EP2017/057693
(87) International publication number: WO 2017/174458

(56) References cited:
- US-A1- 2015 298 984
- JOHAN FORSGREN ET AL: "A Template-Free, Ultra-Adsorbing, High Surface Area Carbonate Nanostructure", PLOS ONE, vol. 8, no. 7, 17 July 2013 (2013-07-17), page e68486, XP055375442, DOI: 10.1371/journal.pone.0068486
- FRYKSTRAND SARA ET AL: "On the pore forming mechanism of Upsalite, a micro- and mesoporous magnesium carbonate", MICROPOROUS AND MESOPOROUS MATERIALS, vol. 190, 19 December 2013 (2013-12-19), pages 99-104, XP028835076, ISSN: 1387-1811, DOI: 10.1016/J.MICROMESO.2013.12.011
- ZHANG PENG ET AL: "Stabilisation of amorphous ibuprofen in Upsalite, a mesoporous magnesium carbonate, as an approach to increasing the aqueous solubility of poorly soluble drugs", INTERNATIONAL JOURNAL OF PHARMACEUTICS, vol. 472, no. 1, 17 June 2014 (2014-06-17) , pages 185-191, XP029037424, ISSN: 0378-5173, DOI: 10.1016/J.IJPHARM.2014.06.025

## Description

### Field of the invention

The present invention relates to a highly porous magnesium carbonate and method of production thereof. The method makes it possible to control the average pore size of the highly porous magnesium carbonate. The method further enables the possibility to adapt the average pore size to a second material, for example a pharmaceutical compound, to be loaded into the highly porous magnesium carbonate.

### Background of the Invention

An important class of nanomaterials is constituted by the micro- and mesoporous materials. Some of the most well-known microporous materials are the zeolites and significant efforts have been spent on developing these crystalline framework materials for gas separation and catalysis. In particular, the pore apertures on some zeolites were found to be adjustable. The pore apertures (or the pore windows) can be controlled by performing different types of post-synthesis treatments, such as cation exchange, heat/vacuum treatment or dehydration.

Whilst the narrow pores of zeolites make them very desirable in applications related to small molecules, a number of different types of mesoporous materials are presently being developed for applications involving larger molecules, including silica, alumina, titania and carbon. Of these, the mesoporous silica materials are the most well-studied and the formation of such materials are well understood and they have reached furthest when it comes to industrial implementation in e.g. chromatography. Good understanding of the reaction mechanism and the final structure are essential for the development of mesoporous materials towards industrial applications. The synthesis of mesoporous silica involves the replication of a surfactant liquid crystal structure and the polymerisation of a silica precursor. Removal of the organic surfactant leads to a porous structure that is supported by a hard silica framework.

Due to its large variation abilities mesoporous silicas have been evaluated in a number of applications ranging from drug delivery, regeneration of bone tissue and as vaccine adjuvants, to catalysis and as sorption media. They can also be functionalised by amine grafting, making them potentially suitable as adsorbents for, e.g., CO₂ separation.

An important aspect regarding the practical usefulness of mesoporous materials is the ability to precisely tailor the pore size to a specific application. Different methods to control the pore size of mesoporous silica have been developed. The methods rely on using specially selected organic template/ surfactants and the addition of a swelling agent in the synthesis or post synthesis. Up to now, accurate pore size control of mesoporous silica can only be achieved with the use of additional organic reagents during the synthesis.

US Patent 9,580,330 discloses a template-free synthesis of a mesoporous magnesium carbonate material with average pore size around 5 nm in diameter. Further investigations disclosed the pore forming mechanism in further detail and it was suggested that the pores were created in a two-step process including the formation of micropores by solvent evaporation and release of physically bound carbon dioxide, followed by micropore-expansion to mesopores due to partial decomposition of organic groups on the surface of the pore walls when the material is stored in air at moderate temperatures. For certain applications, such as drug delivery applications and delivery of other functional agents such as perfumes or nutrients, an increased possibility of tailoring properties of the porous magnesium carbonate, with regards to for example average pore size, would be highly advantageous. Other applications for which pore size control is of interest include separation processes in e.g. the biotech industry or in the oil and gas industry in which e.g. water molecules need to be removed from natural gas to avoid pipeline corrosion, liquid purification in which differently sized entities should be selectively removed from a liquid including water purification as well as in chromatography. With pore size control in such applications one can tailor the pore size of the porous magnesium carbonate in order to optimize the selectivity of the separation or purification process. Likewise, for absorption of odour, caused by e.g. mould, nicotine smoking or fire, tailoring properties of the porous magnesium carbonate is expected to be advantageous for optimizing the absorption properties with respect to the size of the odour molecules to be absorbed by the magnesium carbonate.

For certain applications, for example drug delivery of large molecule compounds, it is advantageous with relatively large mesoproes. US9,580,330 discloses primarily material with pores well below 10 nm, but some samples with higher pore size, around 20 nm, are presented. This prior art material have the drawback of relatively low surface area and low total pore volume.

### Summary of the invention

The possibility of producing a mesoporous material without the use of added template or surfactant where calcination at temperatures as high as those required for making mesoporous silicas are not needed, represents a major breakthrough and may become of high economic importance for future up-scalable production processes of mesoporous materials in a number of applications. This is further emphasized by the findings that very good moisture sorption properties, biocompatibility and drug stabilising properties can be achieved for a highly porous magnesium carbonate made with such synthesis route.

The object of the invention is to provide a production method and a highly porous magnesium carbonate that overcomes the drawbacks of prior art techniques. This is achieved by the material as defined in claim 1 and the method as defined in claim 7. The method enables control of the average pore size of the mesopores of the highly porous magnesium carbonate and makes it possible to provide a material with a combination of large mesopores, high surface area and high total pore volume. The invention further relates to a highly porous magnesium carbonate loaded with, and adapted to, a second material. The second material is typically, but not exclusively, a pharmaceutical compound.

The highly porous magnesium carbonate according to the invention comprises mesopores with an average pore size in the range from 10 nm to 30 nm, and the material has a surface area larger than 120 m²/g as determined by applying the BET method to a nitrogen sorption isotherm and a total pore volume above 0.5 cm³/g as determined by nitrogen sorption isotherms as single point adsorption at relative pressure ∼0.95. The surface area is preferably larger than 150 m²/g, and even more preferably larger than 200 m²/g.

According to one aspect of the invention a combined pharmaceutical compound, or cosmetic product is provided comprising the highly porous magnesium carbonate according to the invention loaded with the pharmaceutical or cosmetic compound. The highly porous magnesium carbonate is particularly suitable for receiving a pharmaceutical compound that is poorly soluble, such as pharmaceutical compounds categorized under the BSC II class.

According to one aspect of the invention the highly porous magnesium carbonate according to the invention is loaded with itraconazole.

According to one aspect of the invention the mesopores have been given a specific average pore size associated with critical confinement dimensions characteristic to the compound or compounds to be loaded into the highly porous magnesium carbonate, the critical confinement dimensions being predetermined to decrease the amorphous to crystalline transition of the compound or compounds. The highly porous magnesium carbonate will act not just as a carrier but will also prevent crystallization.

The method according to the invention of producing a highly porous magnesium carbonate from magnesium oxide, MgO, the highly porous magnesium carbonate comprising mesopores in a range from 2 to 30 nm the method comprising the main steps of:
- sol-gel synthesis comprising mixing magnesium oxide and methanol under CO₂ pressure resulting in a first solution;
- powder formation of the first solution resulting in a wet powder; and
- degassing the wet powder under flow of a non-reactive gas.

The step of powder formation comprises selecting an energy/work input process path based on if large or small average pore volume in the final highly porous magnesium carbonate according is wanted, the selection being low energy/work input process path for large average pore size and high energy/work input process path for small average pore size, and
perfoming the powder formation based on the selection as:
- if low energy/work input process path was selected, enhance the process of CO₂ molecules forming bubbles by decreasing the evaporation rate of CO₂ from the mixture;
- if high energy/work input process path was selected, supress agglomeration of CO₂ molecules into bubbles by means that increases the evaporation rate of CO₂ from the mixture.

The low energy/work input process path represents producing a highly porous magnesium carbonate comprising mesopores in a range from 10 to 30 nm. The high energy/work input process path represents producing a highly porous magnesium carbonate comprising mesopores in a range from 2 to 10 nm.

According to one embodiment of the invention the agglomeration is controlled by selecting a process temperature for the powder formation step from the range -20 to 80 °C. The selected process temperature has a predetermined correspondence to an average pore size. A low process temperatures corresponds to enhanced agglomeration and hence larger average pore size than a high process temperatures.

According to another embodiment the agglomeration is enhanced by providing a mechanically undisturbed environment, and the agglomeration is depressed by subjecting the first solution to mechanical work.

Thanks to the method according to the invention a highly porous magnesium carbonate is provided that offers both comparibly large average pore size, 10-30 nm, and a high surface area, >120 m²/g as determined by applying the BET method to a nitrogen adsorption isotherm.

One advantage of the present invention is that the method is readily up-scaled to industrial processes and no templates or additives are needed for controlling the pore formation.

A further advantage is that the highly porous magnesium carbonate can be tailored to have a specific and predetermined average pore size that is, for example, known to be well suited for receiving a specific drug compound. The highly porous magnesium carbonate can serve both as a drug carrier and to enhance the effect of the drug by hindering crystallization.

### Brief Description of the figures

A more complete understanding of the above mentioned and other features and advantages of the present invention will be apparent from the following detailed description of preferred embodiments in conjunction with the appended drawings, wherein:
Figure 1 is a flowchart illustrating the steps of the method according to the invention;
Figure 2 is a schematic illustration of the composite highly porous magnesium carbonate according to the invention;
Figure 3 is a thermogravimetric curve of transparent the highly porous magnesium carbonate according to the invention;
Figure 4 is a graph showing the particle size of the particles in the reaction mixture after centrifugation and 24 hours of reaction detected by dynamic light scattering;
Figures 5 a-b are graphs showing a) Infrared spectrum of dried transparent highly porous magnesium carbonate according to the invention, and b) Infrared spectrum of the transparent powder heated to 250 °C under nitrogen atmosphere;
Figure 6 shows XPS spectra for Mg₂ₚ and O₁ₛ, for transparent highly porous magnesium carbonate according to the invention, wherein in the Mg₂ₚ spectrum the blue line for the magnesium carbonate fit is completely covered by the black line for the recorded spectrum, indicating an excellent fit;

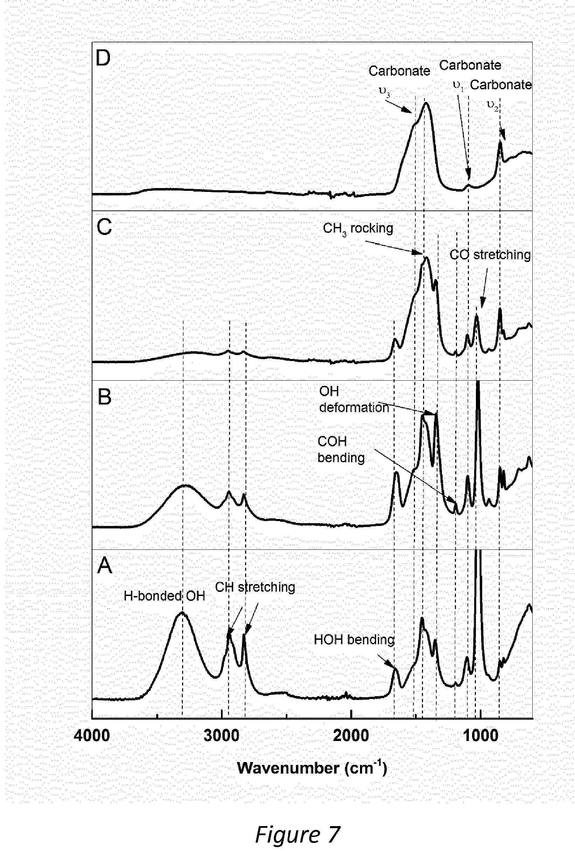

Figures7 a-d are Infrared spectra of a) transparent reaction mixture, b) gel, c) wet powder and d) dried transparent highly porous magnesium carbonate according to the invention;
Figures 8 a-i are (left) N₂ adsorption isotherm and (right) DFT pore size distribution and cumulative pore volume of samples A-G of the highly porous magnesium carbonate according to the invention, the samples are described in Table 2 in the Detailed Description of the Invention;
Figure 9 is a schematic illustration of the pore forming mechanism utilized in the method according to the invention;
Figures 10 a-c are DSC curves of ITZ loaded transparent highly porous magnesium carbonate according to the invention for the three different concentrations of drug loaded into the samples of the highly porous magnesium carbonate according to the invention with varying average pore sizes as well as for the pure drug;
Figure 11 is a graph showing time resolved release in simulated gastric fluid of itraconazole (ITZ) loaded into the highly porous magnesium carbonate according to the invention with average pore sizes of 20 nm (top curve), 13 nm (middle curve) and 5.1 nm (lower curve) as well as dissolution of the pure crystalline drug (bottom curve);
Figure 12a-b are SEM-images illustrating how the amorphous material can collapse to a non-porous material under certain circumstances, wherein b) is an enlargement of a).

### Detailed Description of the Invention

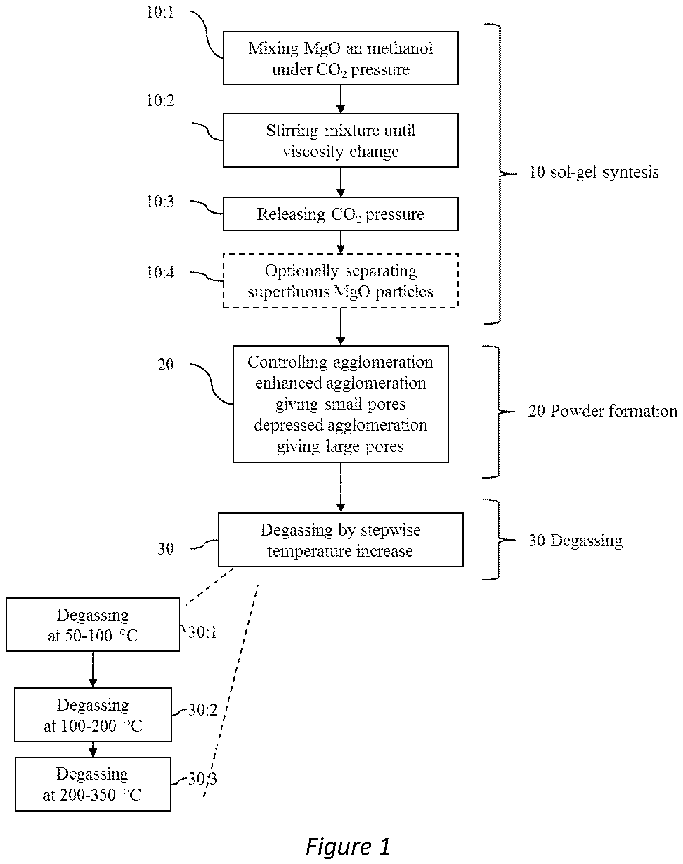

The highly porous magnesium carbonate according to the invention is a composite material that comprises nanometre-sized MgO parts surrounded by amorphous MgCO₃. The amorphous MgCO₃ comprises mesopores of an average pore size in the range ∼2 nm to ∼30 nm. The method according to the invention provides a way to control the average size of the mesopores by controlling the gel/powder formation rate in a powder formation step of the synthesis of the highly porous magnesium carbonate. The highly porous magnesium carbonate according to the invention is synthesised using an optimised version of the sol-gel synthesis method disclosed in the above discussed reference. The method comprises the main steps of i) sol-gel synthesis resulting in a sol from which superfluous MgO particles could be removed by centrifugation, ii) powder formation typically involving stirring that activates gelling and subsequent wet powder generation and finally iii) degassing under nitrogen flow resulting in optically transparent powder particles referred to as transparent highly porous magnesium carbonate. The method will be described with references to the flowchart depicted in Fig. 1:

### 10. Sol-gel synthesis, comprising the steps of:

10.1: mixing magnesium oxide and methanol or ethanol or other alcohols under CO₂ pressure. The CO₂ pressure should be above atmospheric pressure and preferably 1-5 bar.
10.2: stirring the mixture until a change in viscosity can be observed. As realized by the skilled person the mixture may be subjected to other types of mechanically work, such as shaking, tumbling and mixing. A typical process time for this step is in the order of 1 to 10 hours at room temperature.
10.3: releasing CO₂ pressure obtaining a cloudy, yellowish solution.
10.4: optionally separating superfluous MgO particles for example by centrifuging at 5000 rpm (4696g) for 60 minutes to obtain an optically clear, off-white coloured liquid and discarding solid particles. The skilled person may apply other separation methods.

### 20. Powder formation:

The controlling the agglomeration of CO₂ molecules in the above obtained solution into bubbles should be selected in such a way that agglomeration is supressed if the average size of the mesopores of the resulting highly porous magnesium carbonate should be small as compared to the achievable range of mesopores, and the agglomeration is enhanced if the average size of the mesopores should be large as compared to the achievable range of mesopores, the achievable range of mesopores being ∼2 nm-∼30 nm. The agglomeration of CO₂ molecules into bubbles is suppressed by any means that increases the evaporation rate of CO₂ from the mixture, for example increasing the temperature of the mixture or by subjecting the mixture to mechanical work such as, but not limited to, stirring, tumbling or shaking. The agglomeration of CO₂ molecules into bubbles is enhanced by any means that decreases the evaporation rate of CO₂ from the mixture, for example lowering the temperature of the mixture or by providing a mechanically undisturbed environment. The useful temperature range in this step is -20 to 80 °C.

The solution would first thicken into a gel (an alcogel) before breaking up into small, wet powder-like pieces, referred to as wet powder, which is used as an indication that the step is completed.

Powder formation, comprises the steps of:
20:1 Selecting an energy/work input process path based on if large or small average pore size in the final highly porous magnesium carbonate according is wanted. The selection being low energy/work input process path for large average pore size (pore size 10-30 nm), and high energy/work input process path for small average pore size (2-10 nm).
20:2 If low energy/work input process path was selected: Enhance the process of CO₂ molecules forming bubbles by decreasing the evaporation rate of CO₂ from the mixture, for example lowering the temperature of the mixture and/or by providing a mechanically undisturbed environment.
20:3 If high energy/work input process path was selected: Supressing agglomeration of CO₂ molecules into bubbles is by means that increases the evaporation rate of CO₂ from the mixture, for example increasing the temperature of the mixture and/or by subjecting the mixture to mechanical work such as, but not limited to, stirring, tumbling or shaking.

### 30. Degassing, comprising the steps of:

Degassing or drying the wet powder need to be done in a controlled manner to preserve the highly porous structure of the magnesium carbonate. For example, to directly heat at an elevated temperature, typically above 150 °C, would destroy the porous structure and result in a nonporous magnesium carbonate. The degassing is preferably done stepwise, wherein the temperature is increased stepwise and at each temperature degassing is performed until a stable condition is achieved, with regards to the gas given off. The stable condition could be determined by monitoring the weight of the wet powder and not increase the temperature until the weight decrease diminish, observe the rate of the gas given off, or by testing out an appropriate drying scheme by analysing the resulting magnesium carbonate. Alternatively, a continuous increase of the degassing temperature could be utilized, given that the continuous increase is careful enough. Given the knowledge that the degassing needs to be carefully controlled in order to preserve the highly porous nature of the magnesium carbonate, the skilled person may design an appropriate degassing scheme. The degassing should be performed under a slow flow, typically at∼20 cm³/ minute, of a non-reactive gas, i.e. not reacting with the compounds in the wet powder. Nitrogen is a preferred choice of a non-reacting gas.

A preferred degassing scheme comprises the substeps of:
30.1: degassing the wet powder under a slow flow of nitrogen. Typically at ∼20 cm³/ minute at 50-100 °C for at least 1 hour.
30.3: further degassing the wet powder at 100-200 °C for at least 1 hour under a slow flow of nitrogen (∼20 cm³/ minute).

30.3 sub step of a heat treatment and at 200-350 °C for at least 1 hour under a slow flow of nitrogen (∼20 cm³/ minute). The resulting powder is optically transparent and is referred to as "transparent highly porous magnesium carbonate".

In the step of controlling agglomeration (20:1) subjecting the mixture to mechanical work can be done in various ways. In the process described below as a non-limiting example, stirring the solution obtained in the sol-gel synthesis step is done at 60 - 100 rpm in a ventilated area. Appropriate speed and duration will depend on for example size and shape of the reactor vessel, the stirring gear etc. In similar way may the parameters need adjustments if other means of subjecting the solution to mechanical work is used, for example shaking, tumbling, vibrating etc. The skilled persons will, with guidance from the method according to the invention and from the discussion presented below about the agglomeration and pore formation, be able to choose a suitable means for controlling the agglomeration to produce the desired average pore size. Such variations are considered to be within the scope of the present application.

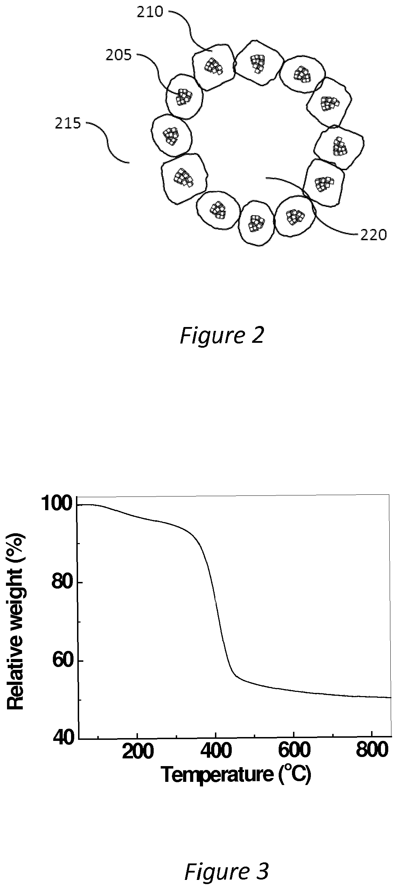

The method according to the present invention makes it possible to produce a composite highly porous magnesium carbonate comprising particles comprising MgO and amorphous magnesium carbonate parts, as shown by DLS, XPS, TGA and elemental analysis, with mesopores whose average size could be controlled from ∼2 nm to ∼30 nm. The composite highly porous magnesium carbonate according to the invention is schematically depicted in Fig. 2. A plurality of nanometre-sized MgO parts 205 and to them associated amorphous magnesium carbonate 210 form ring-like or shell-like structures 215. The mesopores, with a size in the range 10-30 nm, 220 are formed by a plurality of the shell-like structures 215. The mesopores 220 can be seen as remains of the agglomerated CO₂ bubbles discussed in the powder formation step, 20.

The method of the invention may, apart from producing highly porous magnesium carbonate also be used to produce highly porous carbonates of calcium and nickel, or mixtures thereof. As appreciated by the skilled person different starting materials need to be used and adaptions with regards to the process parameters may be required.

The ability to control the pore size of the highly porous magnesium carbonate opens up new possibilities in tailoring the material for certain applications, for examples in applications wherein the highly porous magnesium carbonate is a carrier of a second material or compound, and in applications wherein a certain pore size can be associated to absorption, separation or purification properties. Of particular interest are drug delivery applications wherein the highly porous magnesium carbonate is loaded with a pharmaceutical compound, a drug, or a combination of drugs. Both large and small pores can be of interest.

Certain compounds have enhanced pharmaceutical effect associated with the compound being in an amorphous phase since the *in vivo* bioavailability owing to the low dissolution rate of the crystalline form of the drug is low in the gastrointestinal fluids following oral administration. The transition to a crystalline, and less effective phase, can be hindered or at least delayed, if the compound is confined to dimensions that are characteristic to the drug compound, typically below 10-20 nm. By loading the drug into a highly porous magnesium carbonate which has been given a specific average pore size associated with the characteristic dimensions of the drug compound, the highly porous magnesium carbonate will act as a carrier that prevents crystallisation of the drug. It should be noted that the specific average pore size does not necessarily need to match the size of the drug molecule. Rather, a suitable pore size hindering the crystallisation needs to be determined for a specific drug compound and its intended application, such testing being possible thanks to the method and highly porous magnesium carbonate according to the invention. It is often advantageous to use a material with as large pore size as possible, that still prevents crystallisation, in order to reach high loading degrees. One example, representing one embodiment of the invention, of how to determine a suitable average pore size to prevent crystallisation is given below for the drug itraconazole.

One embodiment of the invention is a highly porous magnesium carbonate wherein the average pore size is adapted to relate to a critical confinement size of a compound to be loaded into the highly porous magnesium carbonate, the confinement size relating to dimensions at which or below crystallisation is hindered or delayed. Drug delivery is an obvious implementation of this embodiment of the invention, but also other applications wherein a transition of the loaded compound can be controlled or hindered by the pore size of the highly porous magnesium carbonate, may be envisaged. A large pore size combined with a large surface area and a high total pore volume is also important for applications utilizing adsorption, for example gas adsorption, and applications utilizing release of active compounds for example fragrances that first are adsorb by the highly porous material and then released or wherein the highly porous material is combined with a surface active agent. One further example is in skin care wherein a cosmetic compound comprising the highly porous magnesium carbonate can be used to absorb excess fat (sebum) from the skin.

A highly porous magnesium carbonate prepared according to the above described method of the invention contained about 10-20 wt.% MgO (and ∼ 80-90 wt.% MgCO₃) as determined by CHN elemental analysis, inductively coupled plasma optical emission spectrometry (ICP-OES) and thermogravimetric analysis (TGA) as illustrated in Fig.3 and table 1. Fig.3 is a graph showing a thermogravimetric curve of transparent highly porous magnesium carbonate, the sample weight at 350 °C and 500 °C were used to back-calculate the MgO content of the starting material. The calculated oxide content using the TGA data was ∼13.8 wt%.This gave a stoichiometric MgO content of the transparent powder of around 30-50 mol%. This portion of MgO could not be removed by centrifugation at 4969 *g*.

**Table 1. Elemental composition and MgO content (from CHN/ICP-OES and TGA) of the transparent highly porous magnesium carbonate, oxygen analysis was not possible using ICP due to the high metal content.**

| | **C** | **H** | **N** | **Mg** | **MgO wt.% (from CHN/ICP)** | **MgO wt.% (from TGA)** |
|---|---|---|---|---|---|---|
| wt. % | 10.80 | 0.28 | <0.01 | 26.2 | 17.6 | 13.8 |

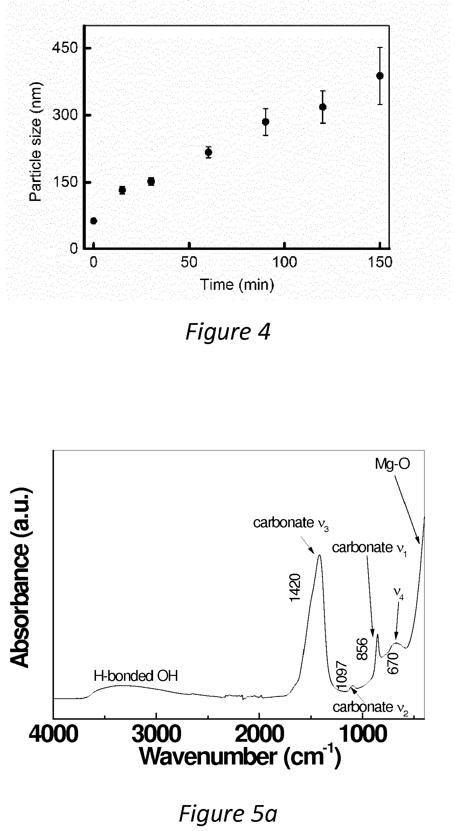

Nanometer-sized aggregates of around 50-100 nm in diameter were detected in the sol described above in the synthesis of highly porous magnesium carbonate using Dynamic Light Scattering (DLS), Fig 4. Significant growth of these nanoparticles occurred with time when the reaction mixture was covered and left standing at room temperature (i.e. without active evaporation/drying). After 2 hours, the nanoparticles became too large to be detected by DLS. The observed particle growth most likely stems from aggregation of particles. CHN analysis, Inductively Coupled Plasma - Optical Emission Spectroscopy (ICP-OES), Thermogravimetric analysis (TGA), X-ray photoemission spectroscopy (XPS) and IR spectroscopy showed that these nanoparticles were composed of MgCO₃ and MgO. Since the highly porous magnesium carbonate is X-ray amorphous, it was concluded that the MgO that is comprised in the material was in the form of small (too small to be detected by powder XRD) nanometer-sized particles surrounded by amorphous MgCO₃. Hence, the highly porous magnesium carbonate can be seen as composite material.

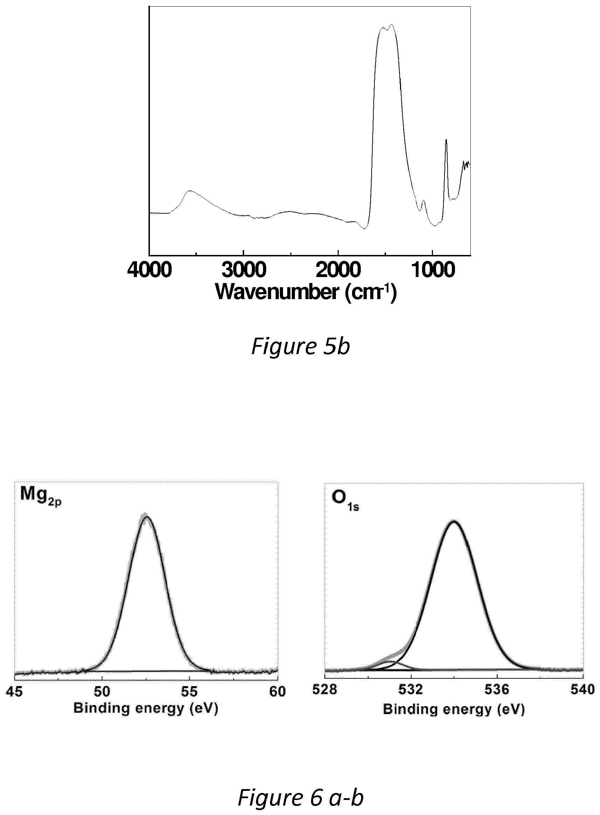

An IR spectrum of the transparent highly porous magnesium carbonate showed that the IR bands related to MgCO₃ were present, see Fig. 5a-b. The Mg₂ₚ and O₁ₛ XPS spectra of the powder, Fig. 6 a-b, showed that the surface of the powder particles contained mainly MgCO₃ and a small amount of MgO. The transparent highly porous magnesium carbonate presented in this study contained a much lower level of MgO than the samples presented in US 9,580,330 due to the optional separating step 10:4.

A discussion of the formation mechanism of the highly porous magnesium carbonate is given below. The discussion is given for illustrative purposes only and should be considered as a non-limiting framework presented in order to further help the skilled person to tailor the material to specific implementations. The results indicate that the material is formed by aggregation of nanometre-sized MgCO₃/MgO composite particles, which will be discussed in detail below. The nanometre-sized composite particles are formed during the reaction between MgO, methanol and CO₂. It has been shown previously that MgO is practically insoluble in methanol (solubility ∼0.1 %). The low solubility of MgO in methanol was also observed in this work, as indicated by an intense white cloudy mixture when the two were mixed. Interestingly, when the reaction vessel was pressurized with CO₂ (4 bar) for 24 hours, the mixture became noticeably less cloudy. The reaction mixture became light yellow and only slightly cloudy, as observed earlier. After centrifugation, the reaction mixture is an optically clear, yellow solution. Tyndall scattering can be observed by shining a red laser light through the solution, indicating the presence of nanoparticles. As explained above, these nanoparticles appeared to be composites of MgO/MgCO₃.

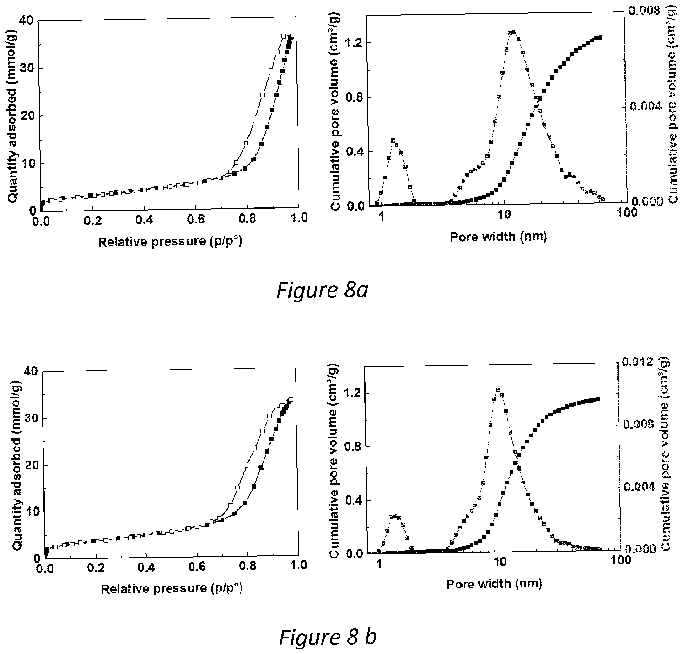

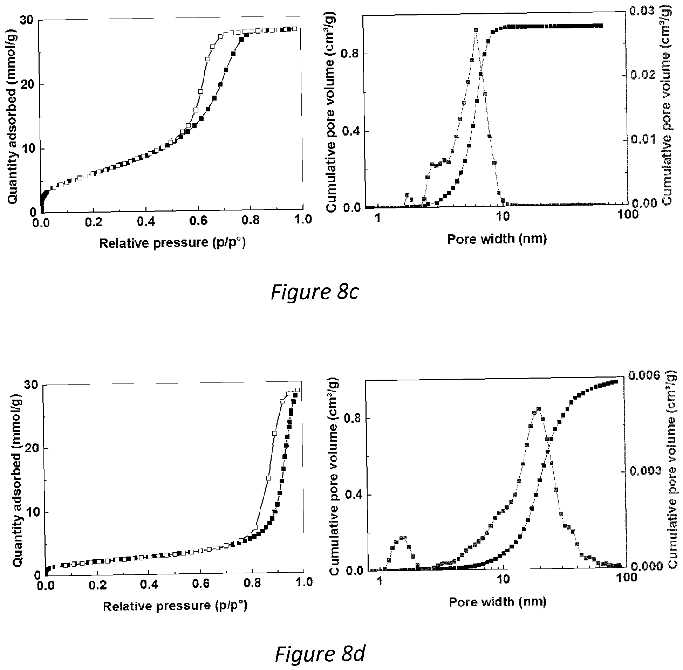

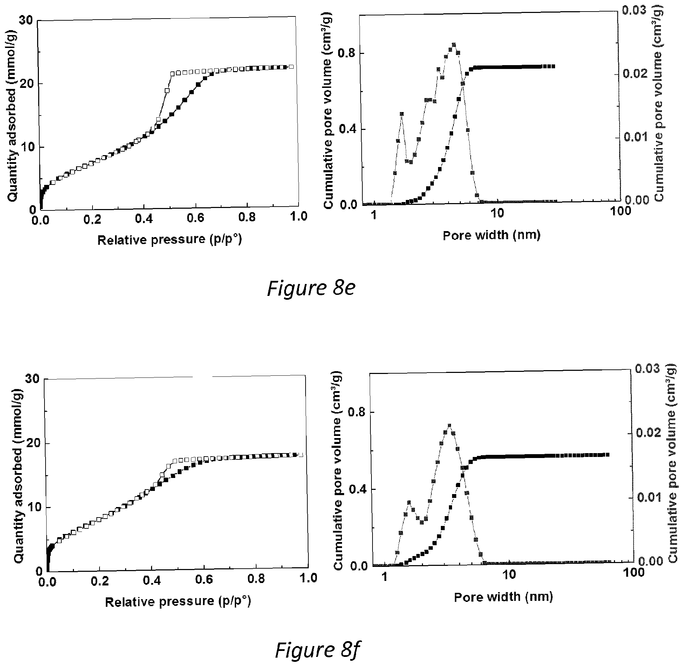

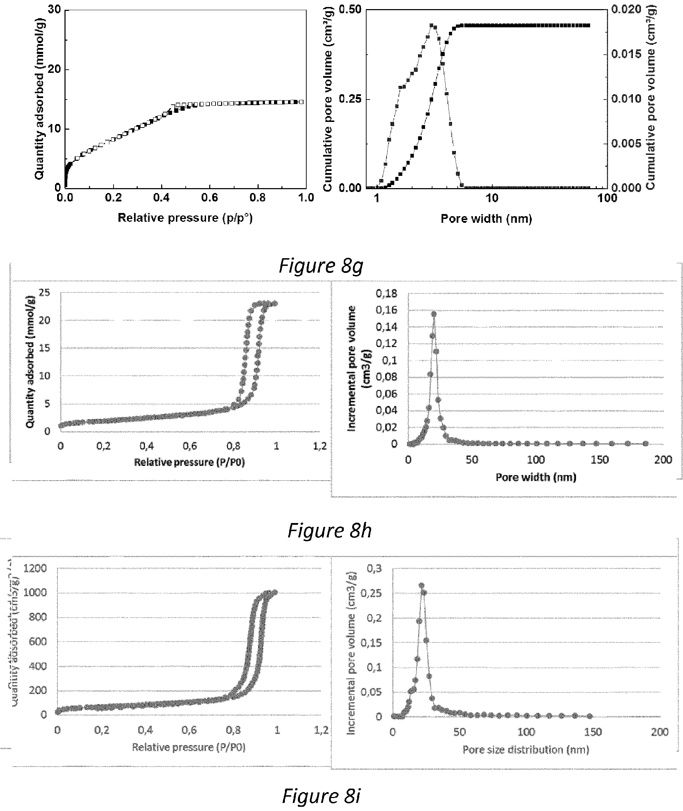

The pore size distributions of materials synthesized using the different powder forming conditions outlined in Table 2 were obtained by density functional theory (DFT) analysis of N₂ sorption isotherms as shown in the graphs of Fig. 8 a-i. The surface area was determined by using the well-recognized BET analysis of nitrogen sorption isotherms. The analysis method hereinafter referred to as the BET method. These transparent samples of highly porous magnesium carbonate were prepared using various powder formation conditions as detailed in Table 2. The table also summarizes the pore properties of the different materials showing that transparent magnesium carbonate powder could be made with very different pore sizes in a controlled manner. Sample I was produced with ethanol as the reaction liquid.

**Table 2. Power formation temperatures, specific surface area and pore properties of different samples. Mechanical stirring was used to form the gel and the powder for all samples except for sample D.**

| **Powder formation temperature** | | | **Peak DFT pore size (nm)** | **Total pore volume (cm³/g)** |
|---|---|---|---|---|
| **Sample** | **(°C)** | **Surface area (m²/g)** | | |
| A | -20 | 263 | ∼13 | 1.27 |
| B | 10 | 297 | ∼10 | 1.15 |
| C | 20 | 499 | ∼6.5 | 0.97 |
| D | 20 (no stirring) | 173 | ∼20 | 1.00 |
| E | 30 | 618 | ∼5.1 | 0.76 |
| F | 50 | 661 | ∼3.4 | 0.60 |
| G | 80 | 690 | ∼2.9 | 0.50 |
| H | RT | 149 | ∼20 | 0.80 |
| I | RT | 225 | ∼22 | 1.55 |

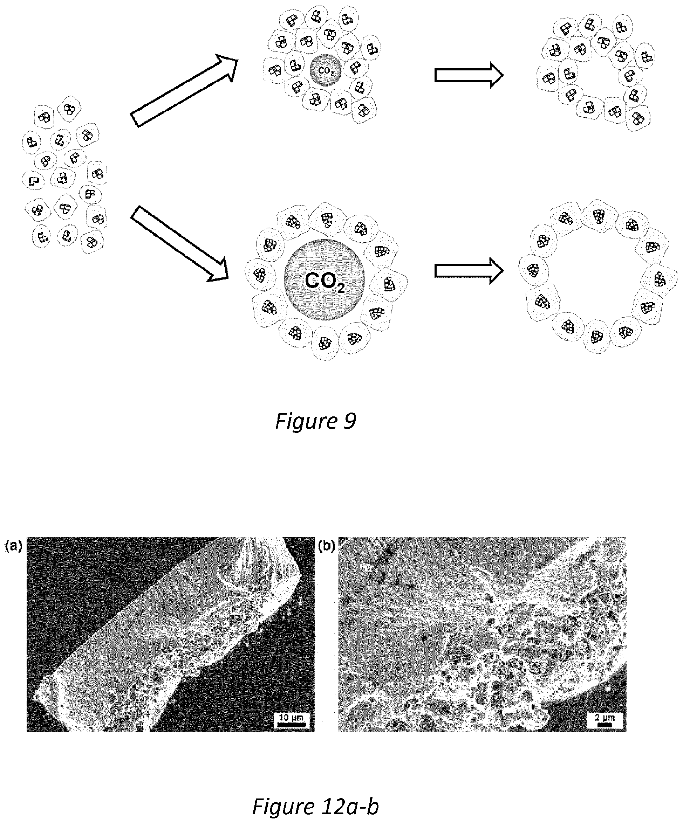

The average size of the mesopores could be controlled from ∼3 nm to -30 nm by adjusting the gel/powder formation rate in the powder formation step, 20 of the synthesis (Fig 1). If the low energy/work input process path 20:2 was selected, mesopores could be formed in the range from -10 nm to -30 nm. If the approach of low energy/work input is taken to the extreme, i.e. leaving the sol-gel solution in step 20 undisturbed for a long time (several days) at a low or moderate temperature the results in a collapse of the porous structure a non-porous X-ray amorphous magnesium carbonate material is formed. Even magnesite (MgCO₃) crystals may form inside the gel. A material with a collapsed porous structure is depicted in Fig. 12a-b.

Fig. 9 is a schematic illustration of how the pores in the material are formed. Fig. 9 also shows how the pore size can be controlled by controlling the agglomeration of CO₂ molecules into bubbles during the powder formation step of the synthesis. During this step, a large amount of CO₂ is given off. The eliminated gas phase CO₂ molecules need to travel to the liquid/air interface between the reaction mixture/gel and ambient air before evaporating from the reaction mixture into gas phase. However, when inside the reaction mixture, the CO₂ molecules aggregate to form bubbles. Nanometre-sized particles (MgCO₃/MgO composites) assemble around these CO₂ bubbles which are then essentially trapped in this configuration. The average size of the bubble renders the average pore size of the material. Low temperature allows CO₂ molecules to form aggregates in the reaction mixture at a higher extent than at high temperature (due to slower kinetics). The slow kinetics results in large bubbles and subsequently large pores. Sample D (Table 2) demonstrated the effect of reduced energy input (by eliminating stirring) clearly, as it has the largest pore size of the synthesized samples in this study. The lower path in the Fig. 9 illustrated the formation process at lower temperatures and the upper path at higher temperatures.

After the wet powder forms, the pores need to be fixed by heating under N₂ flow in a carefully controlled way as described in the degassing step 30. This step fixes the shape of the assembled powder particles and removes the trapped CO₂ bubbles, resulting in a porous solid.

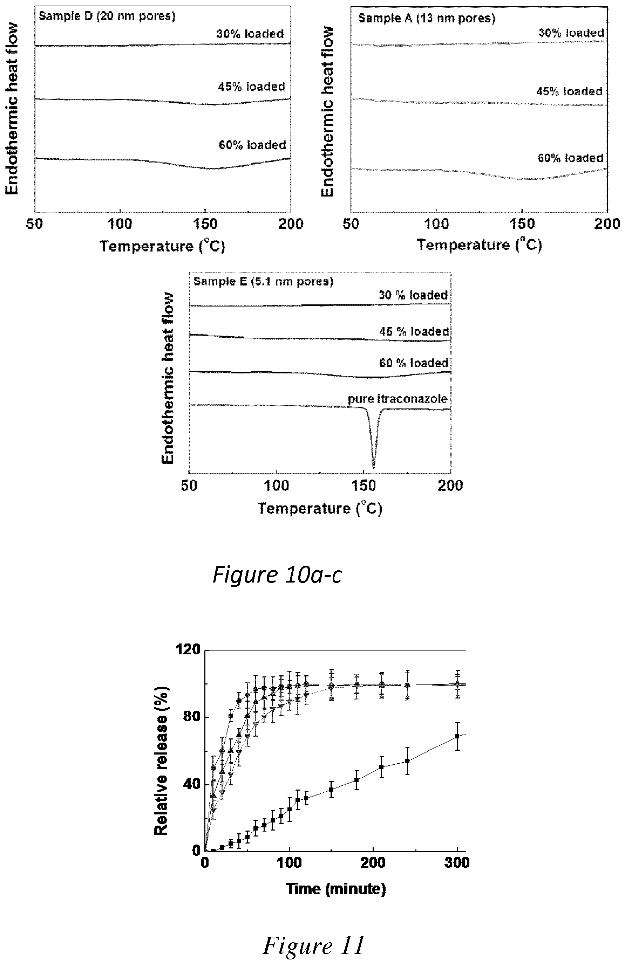

The impact of being able to control pore size of the synthesized material can be shown by its ability to stabilize amorphous compounds and the tailored drug release profile. The drug itraconazole (ITZ) was loaded into transparent highly porous magnesium carbonate samples according to the invention (the drug carrier) with three different average pore sizes (Sample E, A and D with average pore sizes of ∼5.1 nm, -13 nm and -20 nm, respectively). ITZ, in its crystalline form, is a poorly water-soluble antifungal agent Differential scanning calorimetry (DSC) was employed to analyze the structure of ITZ loaded into the pores of the carrier as illustrated in Fig. 10 a-c. TGA was used to confirm the concentration of drug loaded. The described drug loading procedures resulted in the different carrier samples being loaded with 30, 45 and 60 wt% of ITZ. An endothermic peak at 169 °C was observed in the DSC curve for the pure drug. This peak corresponds to the melting point of crystalline ITZ. For all carrier samples loaded with an ITZ concentration corresponding to 30 wt%, this endothermic peak was absent. The lack of such peak confirmed that all carrier samples were able to completely suppress crystallization of the drug loaded inside the pores. The complete lack of endothermic peaks at this degree of loading also indicates that only an insignificant, if any, amount of ITZ resides on the outer surface of the carrier particles. The outer surface area of the carrier particles is negligible compared to the internal surface area and only has a limited ability to interact with the ITZ in order to suppresses the crystallization of the substance. When the drug loading level increased, crystallization was no longer completely suppressed, irrespectively to the sample identity. Whereas samples E (pore size ∼5.1 nm) and A (pore size ∼13 nm) were able to completely stabilize ITZ in the amorphous state at a drug loading degree of 45 wt%, a weak and broad endothermic peak located at the melting point temperature of crystalline ITZ was detected for Sample D (pore size -20 nm). Similar humps were observed for all samples at drug loading degrees of 60 wt%. This illustrates that large pore sizes offer more space for the incorporated drug molecules of small size (having the rectangular cuboid dimensions of 2.97 nm × 0.93 nm × 0.69 nm) to rearrange and crystallize. Furthermore, the finding also emphasized that for optimized stabilization effect of the drug, the pore size of the drug carrier must correlate to the size of the drug. The samples with 5.1 and 13 nm average pores size could host relatively high amounts of ITZ and successfully hindered the crystallization of ITZ (low solubility form of the substance). Since ITZ remained amorphous in all samples at a loading degree of 30 wt%, this degree of drug loading was used for the drug release measurements.

**Table 3. DSC analysis of highly porous magnesium carbonate according to the invention with different average pore sizes loaded with different amounts of itraconazole.**

| | **Average pore size (nm)** | **30 % loading** | **45 % loading** | **60 % loading** |
|---|---|---|---|---|
| **Sample D** | ∼20 | amorphous | Semi -crystalline | Semi-crystalline |
| **Sample A** | ∼13 | amorphous | amorphous | Semi-crystalline |
| **Sample E** | ∼5.1 | amorphous | amorphous | Semi-crystalline |

The time-resolved release of ITZ from the grounded transparent carrier particles into simulated gastric fluid (pH ∼1.3) is shown in Fig. 11, using highly porous magnesium carbonate with average pore sizes of 20 nm (top curve), 13 nm (middle curve) and 5.1 nm (lower curve) as well as dissolution of the pure crystalline drug (bottom curve). The lines are drawn as guides for the eye. The error bars signify variations over three measurements. As observed in the figure, the release rate during the first 30 minutes can be enhanced with a factor of ∼ 23 (release from Sample D as compared to pure ITZ) when ITZ was loaded into the transparent highly porous magnesium carbonate. The corresponding release rate enhancement for samples A and E were ∼17 and ∼13, respectively. During the first 30 minutes of release, no visible changes to the powder in the dissolution vessel were observed.

After a few hours, powder dissolution was observed. This was expected due to the acidic conditions in the release medium. The results showed clearly that the amorphous phase stabilization properties, as well as the release rate, can be tuned by adjusting the pore size of the carrier. This finding opens up for new possibilities to stabilize the vast number of amorphous compounds which are utilized as drugs or potential drug candidates. It should be noted that both MgCO₃ and MgO are GRAS (Generally Recognized as Safe) listed by the U.S. Food and Drug Administration and have E-numbers (E504 for magnesium carbonate and E530 for magnesium oxide).

In many applications, medical as well as other, a large pore size, i.e. larger than 10 nm, together with a high surface area and the possibility to receive a high percentage of the substance to be loaded in the porous material, is highly sought for. The highly porous magnesium carbonate according to the invention with pore size in the range 10 nm to 30 nm and with a surface area larger than 120 m²/g and a total pore volume larger than 0.5 cm³/g, combine providing large pores with the ability of a loading degree, samples A, B, D, H, I. For example, sample A with a surface area of 263 m²/g, a pore size of 13 nm and an initial total pore volume of 1.27 cm³/g, had a remaining total pore volume of 0.59 cm³/g, or 54%, after being loaded with 30 wt% itraconazole. As a comparison a highly porous magnesium carbonate prepared according to US 9,580,330 with 13 nm pores, a surface area of 77 m²/g, and an initial total pore volume of 0.46 cm3/g, had a total pore volume of 0.20 cm3/g, or 43% after being loaded with 30 wt% itraconazole.

In one embodiment of the invention the method of producing the highly porous magnesium carbonate comprising mesopores is optimized for resulting in large pores, i.e. 10-30 nm and a surface area >120 m²/g and a total pore volume >0.5 cm³/g, the surface area and total pore volume determined from nitrogen sorption isotherms. In the powder formation step, step 20, the agglomeration of CO₂ molecules into bubbles is enhanced by any means that decreases the evaporation rate of CO₂ from the mixture, for example lowering the temperature of the mixture or by providing a mechanically undisturbed environment, or with very little disturbance such as gentle stirring. Given the knowledge of how energy input in any form (heating, mechanical work etc) affect the pore size, the skilled person is capable of designing the process step of powder formation to achieve the intended result.

An embodiment of the invention is a highly porous magnesium carbonate comprising mesopores with an average size in the range of 10-30 nm and surface area larger than 120 m²/g, more preferably the surface area is larger than 150 m²/g, and even more preferably larger than 200 m²/g, a total pore volume larger than 0.5 cm³/g, the surface area and the total pore volume determined from nitrogen adsorption isotherms.

The large pore material according to the invention is particularly interesting as a carrier for pharmaceutical compounds displaying poor solubility, for example drugs categorized in the BCS (Biopharmaceutics Classification System) class II. BCS was introduced as a classification system in 2000, it classifies drug substances based on permeability and solubility. BCS class II are substances with high permeability (>90 % after administrated dose) and low solubility (the highest dose can not be dissolved in 250 ml water at pH 1-7.5 and 37 C).

According to one embodiment of the invention a combined pharmaceutical compound and a drug carrier comprising the highly porous magnesium carbonate is provided. For poorly soluble pharmaceutical compounds or compounds belonging to the BCS class II, the highly porous magnesium carbonate should preferably be the large pore material with average pore size in the size range 10-30 nm. A larger pore size is associated with a larger pore volume, which enables a higher loading degree.

According to one embodiment of the invention a cosmetic compound is provided comprising the highly porous magnesium carbonate according to the invention. The highly porous magnesium carbonate is provided to absorb excess fat, sebum, from the skin.

Absorption of excess products from the body could be envisaged also for medical purposes, for example in wound healing, wherein the highly porous magnesium carbonate is provided to absorb for example pus and/or scab. According to one embodiment of the invention a medical compound is provided comprising the highly porous magnesium carbonate according to the invention.

## Claims

1. A highly porous magnesium carbonate comprising mesopores, **characterized in that** the average pore size of the mesopores is in the range from 10 nm to 30 nm, and the material has a surface area larger than 120 m²/g and a total pore volume larger than 0.5 cm³/g, the surface area and the total pore volume determined from nitrogen adsorption isotherms.

2. The highly porous magnesium carbonate according to claim 1, **wherein** the surface area is larger than 150 m²/g, and even more preferably larger than 200 m²/g.

3. The highly porous magnesium carbonate according to claim 1, **wherein** the average pore size of the mesopores is in the range from 13 nm to 22 nm.

4. A combined pharmaceutical compound and drug carrier **characterized by** the drug carrier being the highly porous magnesium carbonate according to claim 1 or 3 loaded with the pharmaceutical compound.

5. The highly porous magnesium carbonate according to claim 4, **wherein** the pharmaceutical compound is poorly soluble or a BSC II class drug.

6. The highly porous magnesium carbonate according to claim 5, **wherein** the pharmaceutical compound is itraconazole.

7. A combined cosmetic compound and carrier **characterized by** the carrier being the highly porous magnesium carbonate according to claims 1 or 3 loaded with the cosmetic compound.

8. A cosmetic compound **comprising** the highly porous magnesium carbonate according to claims 1 or 3, and wherein the highly porous magnesium carbonate is provided to absorb excess fat from the skin.

9. A medical compound **comprising** the highly porous magnesium carbonate according to claims 1 or 3, and wherein the highly porous magnesium carbonate is provided to absorb excess body products, such as pus and/or scab.

10. A method of producing a highly porous magnesium carbonate from magnesium oxide, MgO, the highly porous magnesium carbonate comprising mesopores in a range from 2 to 30 nm the method comprising the main steps of:
- sol-gel synthesis comprising mixing magnesium oxide and methanol under CO₂ pressure resulting in a first solution;
- powder formation of the first solution resulting in a wet powder; and
- degassing the wet powder under flow of a non-reactive gas,
**characterized by that** the step of powder formation comprises selecting an energy/work input process path based on if large or small average pore volume in the final highly porous magnesium carbonate according is wanted, the selection being low energy/work input process path for large average pore size and high energy/work input process path for small average pore size, and
performing the powder formation based on the selection as
- (20:2) if low energy/work input process path was selected, enhance the process of CO₂ molecules forming bubbles by decreasing the evaporation rate of CO₂ from the mixture;
- (20:3) if high energy/work input process path was selected, supress agglomeration of CO₂ molecules into bubbles by means that increases the evaporation rate of CO₂ from the mixture.

11. The method according to claim 10, **wherein** the agglomeration of CO₂ is controlled by selecting a process temperature from the range -20 to 80 °C, and adapting the amount of work depending on the temperature and selected path.

12. The method according to claims 10 or 11, **wherein** the step of sol-gel synthesis comprises separating MgO particles from the first solution prior to the powder formation step.

13. The method according to any of claims 10 to 12, **wherein** the step of degassing comprises a stepwise increase of temperature wherein at each temperature a stable state with regards to gas given of from the powder is achieved before further increase of the temperature.

## Patentansprüche

1. Hochporöses Magnesiumcarbonat, umfassend Mesoporen, **dadurch gekennzeichnet, dass** die durchschnittliche Porengröße der Mesoporen im Bereich von 10 nm bis 30 nm liegt und das Material einen Oberflächenbereich größer als 120 m²/g und ein Gesamtporenvolumen größer als 0,5 cm³/g aufweist, wobei der Oberflächenbereich und das Gesamtporenvolumen aus Stickstoffadsorptionsisothermen bestimmt sind.

2. Hochporöses Magnesiumcarbonat nach Anspruch 1, **wobei** der Oberflächenbereich größer als 150 m²/g und noch mehr bevorzugt größer als 200 m²/g ist.

3. Hochporöses Magnesiumcarbonat nach Anspruch 1, **wobei** die durchschnittliche Porengröße der Mesoporen im Bereich von 13 nm bis 22 nm liegt.

4. Kombinierte pharmazeutische Verbindung und Arzneimittelträger, **gekennzeichnet dadurch, dass** der Arzneimittelträger das hochporöse Magnesiumcarbonat nach Anspruch 1 oder 3 ist, beladen mit der pharmazeutischen Verbindung.

5. Hochporöses Magnesiumcarbonat nach Anspruch 4, **wobei** die pharmazeutische Verbindung schlecht löslich oder ein Arzneimittel der Klasse BSC II ist.

6. Hochporöses Magnesiumcarbonat nach Anspruch 5, **wobei** die pharmazeutische Verbindung Itraconazol ist.

7. Kombinierte kosmetische Verbindung und Träger, **gekennzeichnet dadurch, dass** der Träger das hochporöse Magnesiumcarbonat nach Ansprüchen 1 oder 3 ist, beladen mit der kosmetischen Verbindung.

8. Kosmetische Verbindung, **umfassend** ein hochporöses Magnesiumcarbonat nach Ansprüchen 1 oder 3, und wobei das hochporöse Magnesiumcarbonat bereitgestellt ist, um überschüssiges Fett von der Haut zu absorbieren.

9. Medizinische Verbindung, **umfassend** das hochporöse Magnesiumcarbonat nach Ansprüchen 1 oder 3, und wobei das hochporöse Magnesiumcarbonat bereitgestellt ist, um überschüssige Körperprodukte, wie Eiter und/oder Schorf zu absorbieren.

10. Verfahren zum Herstellen eines hochporösen Magnesiumcarbonats aus Magnesiumoxid, MgO, wobei das hochporöse Magnesiumcarbonat Mesoporen in einem Bereich von 2 bis 30 nm umfasst, wobei das Verfahren die Hauptschritte umfasst:
- Sol-Gel-Synthese, umfassend Magnesiumoxid und Methanol unter CO₂-Druck, was in einer ersten Lösung resultiert;
- Pulverbildung aus der ersten Lösung, was in einem nassen Pulver resultiert; und
- Entgasen des nassen Pulvers unter Fluss eines nicht reaktiven Gases,
**gekennzeichnet dadurch, dass** der Schritt der Pulverbildung Auswählen eines Energie-/Arbeitseinsatzprozesspfads umfasst, darauf basierend, ob großes oder kleines durchschnittliche Porenvolumen in dem endgültigen hochporöses Magnesiumcarbonat gewollt ist, wobei die Auswahl niedrigen Energie-/Arbeitseinsatzprozesspfads für große durchschnittliche Porengröße und hohen Energie-/Arbeitseinsatzprozesspfads für kleine durchschnittliche Porengröße ist, und
Durchführen der Pulverbildung basierend auf der Auswahl als
- (20:2), wenn niedriger Energie-/Arbeitseinsatzprozesspfad ausgewählt wurde, verstärke den Prozess von CO₂-Molekülen, die Blasen bilden, durch Verringern der Verdampfungsrate von CO₂ aus der Mischung;
- (20:3), wenn hoher Energie-/Arbeitseinsatzprozesspfad ausgewählt wurde, unterdrücke Ansammlung von CO₂-Molekülen zu Blasen durch Mittel, die die Verdampfungsrate von CO₂ aus der Mischung steigert.

11. Verfahren nach Anspruch 10, **wobei** die Ansammlung von CO₂ durch Auswählen einer Prozesstemperatur aus dem Bereich -20 bis 80 °C gesteuert wird, und Anpassen der Arbeitsmenge in Abhängigkeit von der Temperatur und dem ausgewählten Pfad.

12. Verfahren nach Ansprüchen 10 oder 11, **wobei** der Sol-Gel-Synthese-Schritt Trennen von MgO-Teilchen aus der ersten Lösung vor dem Pulverbildungsschritt umfasst.

13. Verfahren nach einem der Ansprüche 10 bis 12, **wobei** der Schritt des Entgasens eine schrittweise Erhöhung der Temperatur umfasst, wobei bei jeder Temperatur vor weiterer Erhöhung der Temperatur ein stabiler Zustand hinsichtlich des abgegeben Gases aus dem Pulver erzielt wird.

## Revendications

1. Carbonate de magnésium extrêmement poreux comprenant des mésopores, **caractérisé en ce que** la taille moyenne des pores des mésopores est dans la plage de 10 nm à 30 nm, et le matériau présente une zone de surface plus grande que 120 m²/g et un volume total de pores supérieur à 0,5 cm³/g, la zone de surface et le volume total de pores sont déterminés à partir d'isothermes d'adsorption d'azote.

2. Carbonate de magnésium extrêmement poreux selon la revendication 1, **dans lequel** la zone de surface est plus grande que 150 m²/g, et même de préférence plus grande que 200 m²/g.

3. Carbonate de magnésium extrêmement poreux selon la revendication 1, **dans lequel** la taille moyenne des pores des mésopores est dans la plage de 13 nm à 22 nm.

4. Composé pharmaceutique combiné et support de médicament **caractérisés en ce que** le support de médicament est le carbonate de magnésium extrêmement poreux selon la revendication 1 ou 3 chargé avec le composé pharmaceutique.

5. Carbonate de magnésium extrêmement poreux selon la revendication 4, **dans lequel** le composé pharmaceutique est peu soluble ou un médicament de classe BCS II.

6. Carbonate de magnésium extrêmement poreux selon la revendication 5, **dans lequel** le composé pharmaceutique est de l'itraconazole.

7. Composé cosmétique combiné et support **caractérisés en ce que** le support est le carbonate de magnésium extrêmement poreux selon la revendication 1 ou 3 chargé avec le composé cosmétique.

8. Composé cosmétique **comprenant** le carbonate de magnésium extrêmement poreux selon la revendication 1 ou 3, et dans lequel le carbonate de magnésium extrêmement poreux est fourni pour absorber l'excès de gras de la peau.

9. Composé médical **comprenant** le carbonate de magnésium extrêmement poreux selon la revendication 1 ou 3, et dans lequel le carbonate de magnésium extrêmement poreux est fourni pour absorber l'excès de produits corporels, comme le pus et/ou les croûtes.

10. Procédé de fabrication d'un carbonate de magnésium extrêmement poreux à partir d'oxyde de magnésium, MgO, le carbonate de magnésium extrêmement poreux comprenant des mésopores dans une plage de 2 à 30 nm le procédé comprenant les principales étapes de :
- synthèse de solution-gel comprenant le mélange d'oxyde de magnésium et de méthanol sous pression CO₂ résultant en une première solution ;
- formation d'une poudre de la première solution résultant en une poudre humide ; et
- dégazage de la poudre humide sous un courant gazeux non réactif,
**caractérisé en ce que** l'étape de formation de poudre comprend la sélection d'un circuit de traitement nécessitant de l'énergie/une intervention en fonction du volume grand ou petit de pores moyen dans le carbonate de magnésium extrêmement poreux voulu, la sélection étant un circuit de traitement nécessitant peu d'énergie/intervention pour une taille de pore moyenne grande et un circuit de traitement nécessitant beaucoup d'énergie/intervention pour une taille de pore moyenne petite, et
réalisation de la formation de poudre en fonction de la sélection suivant que
- (20:2) si le circuit de traitement nécessitant peu d'énergie/intervention a été sélectionné, améliore le traitement des molécules de CO₂ en formant des bulles en diminuant le taux d'évaporation du CO₂ du mélange ;
- (20:3) si le circuit de traitement nécessitant beaucoup d'énergie/intervention a été sélectionné, supprime l'agglomération des molécules de CO₂ dans les bulles par des moyens qui augmentent le taux d'évaporation du CO₂ du mélange.

11. Procédé selon la revendication 10, **dans lequel** l'agglomération de CO₂ est contrôlée par la sélection d'une température de traitement dans la plage de -20 à 80 °C, et l'adaptation de la quantité d'intervention en fonction de la température et du circuit sélectionné.

12. Procédé selon la revendication 10 ou 11, **dans lequel** l'étape de synthèse de solution-gel comprend la séparation des particules de MgO de la première solution avant l'étape de formation de poudre.

13. Procédé selon l'une quelconque des revendications 10 à 12, **dans lequel** l'étape de de dégazage comprend une augmentation progressive de la température dans laquelle à chaque température un état stable par rapport au gaz provenant de la poudre est atteint avant d'augmenter davantage la température.
